# EUROPEAN PATENT APPLICATION

(11) **EP 4 803 104 A1**
(43) Date of publication of application: **09.09.2026**
(21) Application number: 24894666.7
(22) Date of filing: 21.11.2024
(51) Int. Cl.: A61K 47/69, A61K 31/713, A61K 48/00, A61P 3/10

(54) **THERAPEUTIC AGENT FOR METABOLIC DISEASES COMPRISING DELIVERY SYSTEM BASED ON METAL NANOPARTICLE-NUCLEIC ACID CONJUGATE**

(30) Priority: 21.11.2023 KR 20230162645
(71) Applicant: NES BIOTECHNOLOGY CO., LTD., Seoul 06974 (KR)
(72) Inventor: LEE, Kangseok, Gwangju-si, Gyeonggi-do 12765 (KR); BAE, Jeehyeon, Gwangju-si, Gyeonggi-do 12765 (KR); YEOM, Jihyun, Yangju-si, Gyeonggi-do 11450 (KR); SIM, Se-hoon, Seoul 08211 (KR); KIM, Hongman, Suwon-si, Gyeonggi-do 16438 (KR); AN, Gyumi, Seoul 06969 (KR); SHIN, Jongeon, Seoul 02577 (KR)
(74) Representative: Fuchs Patentanwälte Partnerschaft mbB
(86) International application number: PCT/KR2024/018566
(87) International publication number: WO 2025/110775

(57) **Abstract**

The present invention relates to a use, as a drug delivery vehicle, of a gene carrier comprising a metal nanoparticle and a double-stranded nucleic acid molecule bound to a surface of the metal nanoparticle, in particular to a use of the carrier for the treatment of a metabolic disease, and to a pharmaceutical composition for the prevention or treatment of a metabolic disease comprising the same.

## Description

### [Technical Field]

The present invention relates to a use, as a drug delivery vehicle, of a carrier comprising a metal nanoparticle and a double-stranded nucleic acid molecule bound to a surface of the metal nanoparticle, and in particular to a therapeutic agent for a metabolic disease comprising the carrier and to a pharmaceutical composition comprising the same.

### [Background Art]

Nanoparticles are particles having a size on the nanometer scale, and possess various physicochemical properties owing to their small size and high surface area. Gold nanoparticles, which are the most widely used nanoparticles, exhibit surface plasmon resonance (SPR) due to absorption and scattering in the visible-light region depending on their size and shape, and are therefore utilized for fluorescence-labeled detection, imaging, and the like; in addition, owing to their ease of introduction of surface functional groups and their high biocompatibility and stability, they can also be used for the delivery of biological substances such as DNA, RNA, proteins, and antibodies, as well as various drugs.

Technologies for delivering genetic material into cells to produce antigens or proteins for therapeutic or prophylactic purposes, such as cell therapy products, are continuously being studied. In particular, vaccine development technologies have grown remarkably as a result of the recent pandemic, and various gene-based vaccines such as DNA vaccines, mRNA vaccines, and viral vector vaccines have been developed.

DNA, as the simplest form of genetic material, is amenable to genetic modification and can therefore shorten the development period when utilized as a therapeutic agent such as a vaccine. Furthermore, compared with other vaccine candidates such as viruses, proteins, and RNA, DNA is highly economical in terms of production facility setup and manufacturing costs, and possesses excellent stability, providing the advantage of being easy to store and distribute. However, DNA vaccines require a process by which the DNA is delivered up to the nucleus of a cell so that mRNA is directly produced within the nucleus. DNA introduced into the nucleus can continuously produce mRNA and steadily produce antigen proteins, but because foreign genetic material is introduced into the cell nucleus, there are concerns regarding side effects such as innate immune responses. In addition, when DNA is delivered via a plasmid, the DNA may, in addition to the antigen, also deliver bacteria-derived genes, which can cause side effects such as mutations within the body. Accordingly, there is a need for research on carriers and delivery technologies for safely delivering DNA into cells.

### [Disclosure of the Invention]

### [Technical Problem]

Accordingly, the present inventors, while diligently conducting research to develop a nucleic acid delivery technology for efficiently delivering, into the nucleus of a cell, a nucleic acid molecule that can be delivered into the cell and independently expressed, in particular a double-stranded DNA (dsDNA), developed a delivery system in which a nucleic acid molecule comprising a gene of interest is directly attached to the surface of a metal nanoparticle through covalent bonding, and have completed the present invention directed to a diabetes therapeutic agent using the same and a pharmaceutical composition for treating a metabolic disease comprising the same.

Therefore, an object of the present invention is to provide a gene carrier for the treatment of a metabolic disease, the gene carrier comprising a gold nanoparticle, and a double-stranded DNA that is bound to the gold nanoparticle through a residue comprising one or more functionalities and, when delivered into a cell, independently expresses insulin or expresses a substance that promotes insulin secretion.

Another object of the present invention is to provide a pharmaceutical composition for the prevention or treatment of a metabolic disease, the pharmaceutical composition comprising the gene carrier.

### [Technical Solution]

In order to achieve the above objects, the present invention provides a gene carrier for the treatment of a metabolic disease, the gene carrier comprising a gold nanoparticle, and a double-stranded DNA that is bound to the gold nanoparticle through a residue comprising one or more functionalities and, when delivered into a cell, independently expresses insulin or expresses a substance that promotes insulin secretion.

In order to achieve another object of the present invention, the present invention provides a pharmaceutical composition for the prevention or treatment of a metabolic disease, the pharmaceutical composition comprising the gene carrier.

Hereinafter, the present invention will be described in detail.

In one aspect, the present invention relates to a gene carrier for the treatment of a metabolic disease, the gene carrier comprising a gold nanoparticle, and a double-stranded DNA that is bound to the gold nanoparticle through a residue comprising one or more functionalities and, when delivered into a cell, independently expresses insulin or expresses a substance that promotes insulin secretion.

The gene carrier of the present invention comprises a metal nanoparticle. The metal nanoparticle has a diameter on the nanometer scale, and is not limited as to its size; preferably, the metal nanoparticle has a diameter of 5 to 500 nm, more preferably 10 to 200 nm. A nanoparticle of this size is easy to prepare in the form of a stable particle, and its size can be readily controlled in the manufacturing process. In addition, in the case of a metal nanoparticle for use as a gene carrier as in the present invention, when the diameter exceeds 500 nm, not only are the properties characteristic of a nanoparticle lost, but also the binding between the metal surface and functional groups becomes weak, presenting the disadvantage that it becomes difficult to prepare a delivery vehicle using the nanoparticle.

In addition, the metal nanoparticle may preferably be a gold nanoparticle, and unlike heavy metals such as manganese, aluminum, cadmium, lead, mercury, cobalt, nickel, and beryllium, the gold nanoparticle is harmless to the human body and thus possesses high biocompatibility.

In one embodiment, the gold nanoparticle used in the present invention may be prepared, for example, as follows: HAuCl₄ is used as a gold source, sodium citrate is used as a reducing agent, and HAuCl₄ is reduced to prepare the gold nanoparticle. In this case, the size of the gold nanoparticle can be controlled by varying the amount of citrate added. That is, as the amount of citrate added increases, nucleation occurs to a greater extent, and accordingly the size of the gold nanoparticle decreases.

The metal nanoparticle of the present invention has a nucleic acid molecule comprising a gene of interest bound to its surface. The nucleic acid molecule is bound to the surface of the metal nanoparticle for the purpose of delivering the gene of interest, which is intended to be introduced into a cell and independently expressed.

The kind of the nucleic acid molecule is not limited. In the present invention, the nucleic acid molecule refers to a compound having a structure in which bases, sugars, and phosphoric acid are linked through phosphodiester bonds, and includes naturally occurring oligonucleotides such as 2'-deoxyribonucleic acid (hereinafter, "DNA") and ribonucleic acid (hereinafter, "RNA"), as well as nucleic acids comprising modified sugar residues, modified phosphate residues, or modified nucleobases. Modifications to the sugar residue include the replacement of the ribose ring with a hexose, cyclopentyl, or cyclohexyl ring. Alternatively, the D-ribose ring of a naturally occurring nucleic acid may be replaced with an L-ribose ring, or the β-anomer of a naturally occurring nucleic acid may be replaced with an α-anomer. The nucleic acid molecule may also comprise one or more abasic moieties. Modified phosphate residues may also include phosphorothioates, phosphorodithioates, methylphosphonates, and methyl phosphates. Such nucleic acid analogs are known to those skilled in the art. A nucleic acid molecule comprising a mixture of two or more of the foregoing may be produced, for example, from a mixture of deoxyribonucleosides or ribonucleosides, in particular from a mixture of deoxyribonucleosides and 2'-O-substituted ribonucleosides such as 2'-O-methyl ribonucleoside or 2'-O-methoxyethyl ribonucleoside.

More specifically, the nucleic acid molecule may be selected from a DNA, an RNA, or a DNA/RNA molecule, and more specifically may be a double-stranded DNA. The double-stranded DNA may comprise cDNA, gDNA, plasmid DNA, and PCR DNA that can be independently expressed. In one embodiment, the double-stranded DNA is bound to the gold nanoparticle in a double-stranded state, in order to deliver the double-stranded DNA into a cell, which differs from the case in which a single-stranded DNA is first bound to a gold nanoparticle and is then introduced into a cell to be hybridized with a complementary strand.

In addition, the nucleic acid molecule may comprise one or more genes of interest and, after being introduced into a cell, may be independently expressed. In the present invention, the term "gene of interest" includes any nucleic acid having a therapeutic, diagnostic, and/or prophylactic effect and/or inducing a desired biological and/or pharmacological effect, and a nucleic acid encoding a functional peptide or polypeptide (protein) of interest (in its native or modified form).

In particular, in the present invention, the gene of interest is one that expresses insulin or a substance that promotes insulin secretion, and is a substance capable of having a prophylactic or therapeutic effect on a metabolic disease through effects such as blood glucose control and appetite suppression. The substance may be, without being limited thereto, the insulin gene (INS, Insulin gene), or, as a substance that promotes insulin secretion, may be selected from GLP-1 (Glucagon-Like Peptide-1), a GLP-1 receptor agonist (GLP-1 Receptor Agonists), GIP (Gastric Inhibitory Polypeptide), oxyntomodulin (Oxyntomodulin), PYY (Peptide YY), CCK (Cholecystokinin), a DPP-4 inhibitor (Dipeptidyl Peptidase-4 Inhibitors), and PDX1 (Pancreatic and Duodenal Homeobox 1).

In the present invention, the term "independently expressed" means that the gene of interest comprised in the nucleic acid molecule undergoes transcription and/or translation by itself, without being integrated into the genome of the cell into which the nucleic acid molecule is introduced. By way of example, but without being limited thereto, in order to be independently expressed, the nucleic acid molecule may comprise one or more promoters, an open reading frame, or a terminator, and more preferably may comprise one or more promoters operably linked to the gene of interest. The promoter sequence is often a eukaryotic promoter derived from or truncated from a virus, and accordingly the promoter may be a pro-opiomelanocortin (POMC) promoter, an adenovirus promoter, a baculovirus promoter, a CMV promoter, a parvovirus promoter, a herpesvirus promoter, a poxvirus promoter, an adeno-associated virus promoter, a Semliki Forest virus promoter, an SV40 promoter, a vaccinia virus promoter, or a retrovirus promoter. Examples of the promoter include the human herpes simplex virus thymidine kinase (HSV TK or miniTK) promoter, the cauliflower mosaic virus (CaMV) 35S promoter, the human cytomegalovirus CMV promoter (miniCMV), CMV53 (a minCMV with an upstream GC box added), the simian virus 40 promoter (minSV40), MLP (the -38 to +6 region of the adenovirus major late promoter), minP (a synthetic promoter consisting of a TATA box and a transcription start site-a Promega product), pJB42CAT5 (a promoter derived from the human *junB* gene), YB_TATA, and the Super Core Promoter 1 (SCP1) promoter. Several promoters (sometimes also referred to as "core promoters") are described in the literature (Ede et al., ACS Synth Biol 2016 May 20; 5(5): 395-404).

The terms "operably arranged," "operably bound," and "operably linked" mean that a promoter (and/or enhancer) is in a precise functional position and orientation with respect to a nucleic acid sequence to control the initiation of transcription and the expression of the nucleic acid. An enhancer is "operably linked" to a promoter when it is in a precise functional position and orientation to increase the transcriptional activity of the promoter.

In one embodiment, the nucleic acid molecule further comprises a polyadenylation (poly(A)) sequence. The poly(A) sequence causes appropriate polyadenylation of the nucleic acid (transcript) of interest. Examples of representative poly(A) sequences include SV40 poly(A) and/or bovine growth hormone poly(A), which are known to function conveniently and/or favorably in various target cells.

In one embodiment, the nucleic acid molecule further comprises a transcription termination sequence. A "termination signal" or "terminator" consists of a DNA sequence that is involved in the specific termination of an RNA transcript by an RNA polymerase.

In addition, without being limited thereto, the nucleic acid molecule can produce a transcription product and/or a translation product through an expression process of being transcribed and/or translated. The transcription product and/or translation product may include, without being limited thereto, for example, mRNA, non-coding RNA, a protein, an antigen, or an antibody.

The gene carrier of the present invention is one in which a nucleic acid molecule is bound to the surface of a gold nanoparticle. The nucleic acid molecule comprises one or more functionalities for binding to the surface of the gold nanoparticle. The kind of the functionality is not limited, and the functionality may be a thiol group or an amine group, and may be comprised in one or more residues of the nucleic acid molecule to be delivered. In one embodiment of the present invention, the nucleic acid molecule comprises one or more thiolated residues, through which it is directly bound to the surface of the gold nanoparticle. The binding does not include a separate spacer or linker. The thiolated residue may be one or more bases at the 3' end, the 5' end, or within the nucleotide sequence of the nucleic acid molecule.

In addition, one or more nucleic acid molecules may be bound to the surface of the gold nanoparticle, and without being limited thereto, 1 to 20 nucleic acid molecules may be bound to the surface of the gold nanoparticle for expression. Furthermore, the length of the bound nucleic acid molecule is at least 100 bp, 200 bp, or 300 bp or more, and is not limited thereto. In some cases, the nucleic acid molecule has more than 30 nucleotides.

In another embodiment, the nucleic acid molecule has more than 35 nucleotides. In another embodiment, the length is at least 40 nucleotides. In another embodiment, the length is at least 45 nucleotides. In another embodiment, the length is at least 55 nucleotides. In another embodiment, the length is at least 50 nucleotides. In another embodiment, the length is at least 60 nucleotides. In another embodiment, the length is at least 80 nucleotides. In another embodiment, the length is at least 90 nucleotides. In another embodiment, the length is at least 100 nucleotides. In another embodiment, the length is at least 120 nucleotides. In another embodiment, the length is at least 140 nucleotides. In another embodiment, the length is at least 160 nucleotides. In another embodiment, the length is at least 180 nucleotides. In another embodiment, the length is at least 200 nucleotides. In another embodiment, the length is at least 250 nucleotides. In another embodiment, the length is at least 300 nucleotides. In another embodiment, the length is at least 350 nucleotides. In another embodiment, the length is at least 400 nucleotides. In another embodiment, the length is at least 450 nucleotides. In another embodiment, the length is at least 500 nucleotides. In another embodiment, the length is at least 600 nucleotides. In another embodiment, the length is at least 700 nucleotides. In another embodiment, the length is at least 800 nucleotides. In another embodiment, the length is at least 900 nucleotides. In another embodiment, the length is at least 1,000 nucleotides. In another embodiment, the length is at least 1,100 nucleotides. In another embodiment, the length is at least 1,200 nucleotides. In another embodiment, the length is at least 1,300 nucleotides. In another embodiment, the length is at least 1,400 nucleotides. In another embodiment, the length is at least 1,500 nucleotides. In another embodiment, the length is at least 1,600 nucleotides. In another embodiment, the length is at least 1,800 nucleotides. In another embodiment, the length is at least 2,000 nucleotides. In another embodiment, the length is at least 2,500 nucleotides. In another embodiment, the length is at least 3,000 nucleotides. In another embodiment, the length is at least 4,000 nucleotides. In another embodiment, the length is at least 5,000 nucleotides, or exceeds 5,000 nucleotides.

In another aspect of the present invention, the present invention relates to a pharmaceutical composition comprising the gene carrier comprising the gold nanoparticle and the double-stranded DNA. The pharmaceutical composition is for the prevention, alleviation, or treatment of a disease, is not limited as to its kind, and may have different uses depending on the kind of double-stranded DNA bound to the gold nanoparticle and the expression product thereof.

In particular, the pharmaceutical composition of the present invention may be a prophylactic or therapeutic agent for a metabolic disease, and the metabolic disease, which is a disease caused by a problem occurring in the body's metabolic processes, may include diabetes, obesity, dyslipidemia, fatty liver disease, metabolic syndrome, or cardiovascular disease such as hypertension, and may preferably be diabetes, but is not limited thereto.

The pharmaceutical composition may be a cell and gene therapy product comprising a cell therapy product, a genetically modified cell therapy product, a gene therapy product, or an RNA therapeutic agent.

The cell therapy product refers to a pharmaceutical product that is used for the purposes of treatment, diagnosis, and prevention through a series of actions such as proliferating and selecting *ex vivo* living autologous, allogeneic, or xenogeneic cells, or otherwise altering the biological characteristics of cells, in order to restore the tissues and functions of cells; in the case of modifying intracellular genes, it is also classified as a genetically modified cell therapy product. In addition, the gene therapy product is a pharmaceutical product manufactured for the purpose of treating or preventing a gene defect by introducing normal genes and therapeutic genes into the cells of a patient using genetic manipulation such as genetic recombination, thereby correcting defective genes or adding new functions to the cells.

Furthermore, the RNA therapeutic agent exerts drug efficacy by inhibiting a process of producing a disease-inducing protein from a target gene, and may include mRNA, RNAi, ASO (antisense oligonucleotide), an RNA aptamer, and the like.

The pharmaceutical composition according to the present invention may further comprise an appropriate carrier, excipient, and diluent commonly used in the preparation of a pharmaceutical composition. The excipient may be, for example, one or more selected from the group consisting of a diluent, a binder, a disintegrant, a lubricant, an adsorbent, a humectant, a film-coating material, and a controlled-release additive.

The pharmaceutical composition according to the present invention may be formulated and used in the form of, according to conventional methods, powders, granules, sustained-release granules, enteric granules, solutions, eye drops, elixirs, emulsions, suspensions, spirits, troches, aromatic waters, lemonades, tablets, sustained-release tablets, enteric tablets, sublingual tablets, hard capsules, soft capsules, sustained-release capsules, enteric capsules, pills, tinctures, soft extracts, dry extracts, fluid extracts, injections, capsules, perfusates, plasters, lotions, pastes, sprays, inhalants, patches, sterile injectable solutions, or external preparations such as aerosols, and the external preparation may have a formulation such as a cream, a gel, a patch, a spray, an ointment, a plaster, a lotion, a liniment, a paste, or a cataplasma.

The carrier, excipient, and diluent that may be comprised in the pharmaceutical composition according to the present invention include lactose, dextrose, sucrose, oligosaccharides, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, and mineral oil.

When formulated, the pharmaceutical composition is prepared using diluents or excipients commonly used, such as fillers, bulking agents, binders, wetting agents, disintegrants, and surfactants.

The pharmaceutical composition according to the present invention is administered in a pharmaceutically effective amount. In the present invention, "pharmaceutically effective amount" means an amount sufficient to treat a disease at a reasonable benefit/risk ratio applicable to medical treatment, and the effective dose level may be determined according to factors well known in the medical art, including the kind and severity of the disease of the patient, the activity of the drug, sensitivity to the drug, time of administration, route of administration and excretion rate, duration of treatment, drugs concurrently used, and other factors. The pharmaceutical composition according to the present invention may be administered as a single therapeutic agent or in combination with other therapeutic agents, may be administered sequentially or simultaneously with conventional therapeutic agents, and may be administered in a single dose or in multiple doses. Taking into consideration all of the foregoing factors, it is important to administer an amount that achieves the maximum effect with a minimum amount and without side effects, which can be readily determined by a person having ordinary skill in the art to which the present invention pertains.

The pharmaceutical composition of the present invention may be administered to a subject by various routes. All modes of administration are contemplated; for example, administration may be carried out by oral administration, subcutaneous injection, intraperitoneal administration, intravenous injection, intramuscular injection, intrathecal injection (into the space surrounding the spinal cord), sublingual administration, buccal administration, rectal insertion, vaginal insertion, ocular administration, otic administration, intranasal administration, inhalation, spraying through the mouth or nose, cutaneous administration, transdermal administration, or the like.

The pharmaceutical composition of the present invention is determined depending on the kind of drug as the active ingredient together with various related factors such as the disease to be treated, the route of administration, and the age, sex, and body weight of the patient and the severity of the disease. In the present invention, "subject" refers to a subject in need of treatment of a disease, and more specifically refers to mammals such as humans or non-human primates, mice, rats, dogs, cats, horses, and cattle.

In the present invention, "administration" means providing a subject with a predetermined composition of the present invention by any suitable method.

In the present invention, "prevention" refers to any action that suppresses or delays the onset of the metabolic disease of interest; "treatment" refers to any action by which the metabolic disease and the metabolic abnormality symptoms accompanying it are alleviated or beneficially altered through the administration of the pharmaceutical composition according to the present invention; and "alleviation" refers to any action that reduces a parameter related to the disease of interest, for example the degree of symptoms, through the administration of the composition according to the present invention.

In addition, in another aspect of the present invention, the present invention relates to a method for preparing a gene carrier, the method comprising: a step of treating a metal nanoparticle with an acidic solution to modify the surface of the metal nanoparticle; and a step of binding to the surface of the metal nanoparticle a nucleic acid molecule comprising one or more genes of interest that are delivered into a cell and expressed.

In addition, in yet another aspect of the present invention, the present invention relates to a method for expressing a gene that expresses insulin or that promotes insulin secretion, through a nucleic acid molecule that is bound to the surface of a metal nanoparticle and is independently expressed within a cell.

### [Advantageous Effects of the Invention]

The present invention relates to a gene carrier comprising a metal nanoparticle and a nucleic acid molecule comprising a gene of a substance that expresses insulin or promotes insulin secretion bound to the surface of the metal nanoparticle, to a method for preparing the same, and to uses thereof; in particular, a nucleic acid molecule in the form of a double-stranded DNA is directly bound to the surface of a gold nanoparticle through a covalent bond and delivered into a cell to be expressed, thereby enabling stable gene delivery and expression, and in particular, by expressing insulin or a substance that promotes insulin secretion, the present invention can be utilized as a pharmaceutical composition for the prevention or treatment of a metabolic disease.

### [Brief Description of the Drawings]

FIG. 1 is a schematic diagram showing the structure of a nucleic acid molecule comprising a gene of interest bound to a gold nanoparticle in one embodiment of the present invention.
FIG. 2 is a schematic diagram of insulin and Bioactive (BA) insulin used in one embodiment of the present invention.
FIG. 3 shows expression systems of insulin and Bioactive (BA) insulin constructed in one embodiment of the present invention.
FIG. 4 shows the result of confirming intracellular expression using the expression systems constructed in the present invention.
FIG. 5 shows the result of confirming the binding efficiency of nucleic acid molecules bound to the gold nanoparticle of the present invention through thiolated residues.
FIG. 6 shows the result of confirming gene delivery and expression in a small animal model of a gold nanoparticle carrier loaded with double-stranded DNA.

### [ Mode for the Invention]

Hereinafter, the present specification will be described in detail with reference to Examples to specifically explain the present specification. However, the Examples according to the present specification can be modified into various different forms, and the scope of the present specification should not be construed as being limited to the Examples described in detail below. The Examples of the present specification are provided to more completely explain the present specification to a person having ordinary skill in the art.

### Example 1. Preparation of Double-Stranded DNA for Insulin Expression

### 1-1. Preparation of Plasmids for Intracellular Insulin Expression

The INS gene was synthesized by PCR using primers 1 and 2 from cDNA of pancreatic beta cells, and then inserted between the CMV promoter and the bGH terminator of pcDNA3.1 by the In-Fusion method to construct pNES1-INS (SEQ ID NO: 12).

Mutations were introduced into the INS gene by the PCR method using primers of SEQ ID NOs: 3 and 4, SEQ ID NOs: 5 and 2, and SEQ ID NOs: 3 and 2, and the resulting fragment was inserted between the CMV promoter and the bGH terminator of pcDNA3.1 by the In-Fusion method to construct pNES1-BA-INS (SEQ ID NO: 13).

The region from the CMV promoter to the beta-globin polyA signal of pAAV2-CMV was amplified by PCR using primers of SEQ ID NOs: 6 and 7, and the resulting sequence was substituted, by the In-Fusion method, for the sequence from the CMV promoter to the bGH terminator of pcDNA3.1, thereby constructing pNES3.

In addition, after PCR amplification from pNES1-INS and pNES1-BA-INS using primers of SEQ ID NOs: 8 and 9, the amplified fragments were inserted between the beta-globin intron and the beta-globin polyA of pNES3 by the In-Fusion method to construct pNES3-INS (SEQ ID NO: 14) and pNES3-BA-INS (SEQ ID NO: 15). The primer sequences used in the foregoing are shown in Table 1 below.

**[Table 1]**

| **SEQ ID NO** | **Name** | **Sequence** |
|---|---|---|
| 1 | pNES1-INS-SP F | AACTAGAGAACCCACTGCTTACTGGCGCCGCCACCATGGCC CTGTGGATGCGCCTCCTGCCCCTGCTGGCGCTGC |
| 2 | pNES1-INS R | CAGATGGCTGGCAACTAGAAGGCACAGTCGCTAGTTGCAGT AGTTCTCCAG |
| 3 | pNES1-INS F | AACTAGAGAACCCACTGCTTACTGGCGCCGCCACCATGGCC CTGTGGATGCGCCTC |
| 4 | BA-INS R | AGGGCCCCCGCCCAGCTCCACCTGCCCCACCTGCAGGTCCTC TGCCTCCCGcttGGTCcTGGGTGTGTAGAAGAAGCCTCG |
| 5 | BA-INS F | GTGGAGCTGGGCGGGGGCCCTGGTGCAGGCAGCCTGCAGC CCTTGGCCCTGGAGGGGTCCCgGCAGAAGCGTGGCATTGTG GAA |
| 6 | CMV-F | CAGATATACGCGTTGACATTGATTATTGACTAGTT |
| 7 | b-globin PA-R | TTTCCGCCTCAGAAGAAAATACAGCATAGCAAAAC |
| 8 | pNES3-INS F | GATCCACCGGTCGCCACCATGGCCCTGTGGATGCGC |
| 9 | pNES3-INS R | TAAGCTTGGATCCTCTAGAGCGGCCGCTCTAGTTGCAGTAG TTCTCCAGCTG |

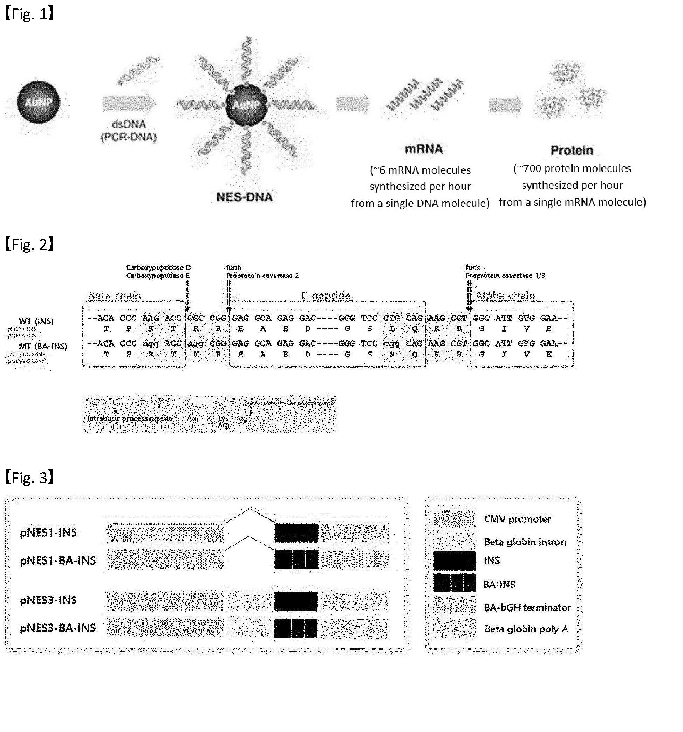

The plasmids constructed as a result of the foregoing are shown in FIG. 3.

### 1-2. Confirmation of Intracellular Expression of Insulin and BA-Insulin Using the Plasmids

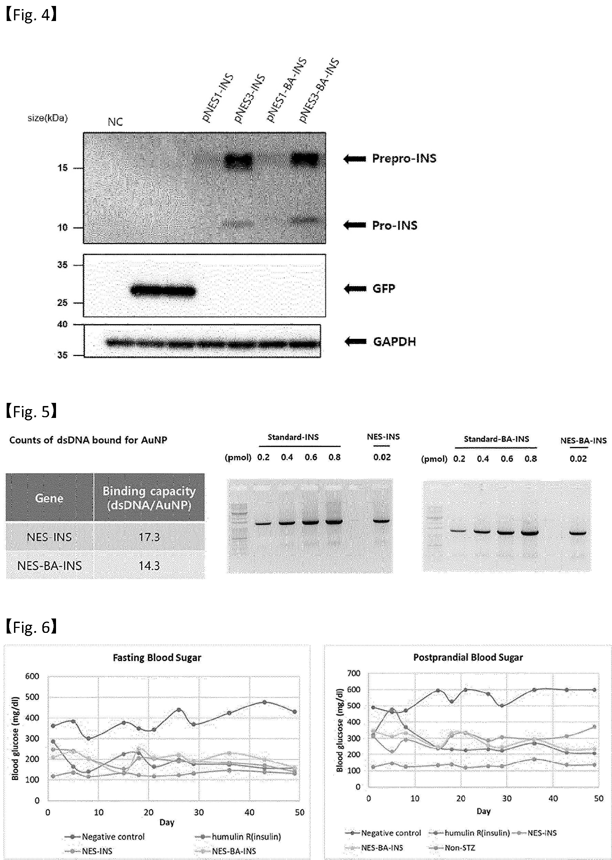

Expression of the plasmids comprising the INS and BA-INS genes constructed above was carried out using a cell line (HeLa cells). 1 µg of plasmid was delivered into cells in a culture plate in which 3 × 10⁵ cells were cultured, using Lipofectamine 3000, and insulin expression was observed by Western blot. As a result, insulin expression was confirmed in all of pNES1-INS, pNES1-BA-INS, pNES3-INS, and pNES3-BA-INS (FIG. 4).

### Example 2. Preparation of Gold Nanoparticles Expressing Insulin

Thiolated double-stranded DNA for intracellular expression was bound to gold nanoparticles to prepare AuNP-dsDNA capable of intracellular delivery and expression by the following procedure, of which a schematic diagram is shown in FIG. 1.

### 2-1. Preparation of dsDNA Capable of Intracellular Antigen Expression

As the gene to be delivered, the INS and BA-INS genes were cloned by the In-Fusion method as described above, and in order to thiolate the 5'-end residues, the 5'-end thiolation primer sequences shown in Table 2 below were used to synthesize thiolated double-stranded DNAs of INS and BA-INS.

**[Table 2]**

| **SEQ ID NO** | **Name** | **Sequence** |
|---|---|---|
| 10 | pcDNA3.1 dsDNA F | 5' - (Thiolation) C*T*T*A*G*GGTTAGGCGTTTTGC |
| 11 | pcDNA3.1 dsDNA R | 5' - C*T*A*C*A*GGGCGCGTGGGGATAC |

| | | |
|---|---|---|
| *Phosphorothioate backbone | | |

### 2-2. Pretreatment of Thiolated Double-Stranded DNA

The synthesized thiolated double-stranded DNA was dissolved in water to a final concentration of 1 µM, and then 20 µL of 3 M sodium acetate (pH 5.2) and 30 µL of 1 N DTT (dithiothreitol) were added to 150 µL of the thiolated double-stranded DNA, and the mixture was reacted at room temperature for 60 minutes. To remove the DTT containing unwanted thiol-group molecules, 200 µL of ethyl acetate was added and mixed, followed by centrifugation to remove the supernatant; this procedure was repeated three times. Subsequently, the thiolated double-stranded DNA was precipitated using the ethanol (EtOH) precipitation method.

### 2-3. Preparation of dsDNA-Functionalized Gold Nanoparticles (AuNP-dsDNA)

The thiolated double-stranded DNA pretreated and precipitated through the procedure of Section 2-2 above was dissolved in water, added to gold nanoparticles, and then bound thereto by the salt aging method. Specifically, the thiolated double-stranded DNA was added to 7 nM gold nanoparticles (AuNP : thiolated double-stranded DNA = 1:40), thoroughly mixed, and NaCl was then added so that the concentration was 0.1 M, followed by mixing for 4 hours. After 4 hours, NaCl was added so that the concentration was 0.2 M, followed by mixing for 4 hours. After 4 hours, NaCl was added so that the concentration was 0.3 M, followed by mixing for 12 hours.

After 12 hours, the mixture of the thiolated double-stranded DNA and the gold nanoparticles was collected by centrifugation at ~10,000 × g for 20 minutes, and the unreacted double-stranded DNA in the supernatant was removed; this procedure was repeated three times.

The final AuNP-thiolated double-stranded DNA conjugate (AuNP-thiolated dsDNA) was dispersed in 10 mM sodium phosphate buffer (pH 7.4) containing 0.1 M NaCl. The prepared AuNP-thiolated double-stranded DNA conjugate was analyzed by electrophoresis on a 10% acrylamide / 8 M urea gel, and it was confirmed that 1.98 to 9.27 thiolated double-stranded DNAs were bound per gold nanoparticle (FIG. 5).

### Example 3. Confirmation of Gene Delivery and Expression by AuNP-dsDNA (Insulin) in a Small Animal Model

As the diabetes model mouse experiment, a Type 1 diabetes model induced by drug induction with streptozotocin (STZ) was used. Diabetes was induced in 8-week-old BALB/c female mice (Central Lab Animal Inc., Korea) by treatment with STZ at 50 mg/kg for 5 days, and AuNP-thiolated dsDNA (insulin) was injected once into the thigh muscle of the mice, and changes in blood glucose were monitored for 50 days. As a result, it was confirmed that NES-INS and NES-BA-INS reduced blood glucose in the diabetes model mice to a level similar to that of mice receiving daily insulin injections (FIG. 6).

Heretofore, the present invention has been described with a focus on preferred embodiments thereof. A person having ordinary skill in the art to which the present invention pertains will appreciate that the present invention may be embodied in modified forms without departing from the essential characteristics thereof. Therefore, the disclosed embodiments should be considered in an illustrative rather than a restrictive sense. The scope of the present invention is set forth in the claims rather than in the foregoing description, and all differences within the scope equivalent thereto should be construed as being included in the present invention.

### [Mode for Carrying Out the Invention]

In one aspect, the present invention relates to a gene carrier comprising a gold nanoparticle, and a double-stranded DNA that is bound to the gold nanoparticle through a residue comprising one or more functionalities and, when delivered into a cell, independently expresses insulin or expresses a substance that promotes insulin secretion.

In one embodiment, the double-stranded DNA comprises an insulin gene (INS, Insulin gene).

In one embodiment, the substance that promotes insulin secretion comprises one selected from GLP-1 (Glucagon-Like Peptide-1), a GLP-1 receptor agonist (GLP-1 Receptor Agonists), GIP (Gastric Inhibitory Polypeptide), oxyntomodulin (Oxyntomodulin), PYY (Peptide YY), CCK (Cholecystokinin), a DPP-4 inhibitor (Dipeptidyl Peptidase-4 Inhibitors), and PDX1 (Pancreatic and Duodenal Homeobox 1).

In one embodiment, the double-stranded DNA can be independently expressed without being integrated into the genome of the cell into which the double-stranded DNA is introduced.

In one embodiment, the residue comprising the functionality comprises a thiol group or an amine group, and the residue comprising the functionality may be one comprised at the 3' end, the 5' end, or within the nucleotide sequence of the double-stranded DNA.

In one embodiment, the gold nanoparticle has a size of 5 to 500 nm.

In another aspect, the present invention relates to a pharmaceutical composition for the prevention or treatment of a metabolic disease, the pharmaceutical composition comprising the gene carrier.

In one embodiment, the metabolic disease may be a disease selected from the group consisting of diabetes, obesity, dyslipidemia, fatty liver disease, metabolic syndrome, and cardiovascular disease such as hypertension.

In yet another aspect, the present invention relates to a method for the prevention or treatment of a metabolic disease using a gold nanoparticle carrier, the method comprising: a step of binding, to a surface of a gold nanoparticle, a double-stranded DNA that expresses insulin or that expresses a substance that promotes insulin secretion, and introducing the same into a cell; and a step of independently expressing, within the nucleus, the double-stranded DNA bound to the surface of the gold nanoparticle.

In yet another aspect, the present invention relates to a use, for the prevention or treatment of a metabolic disease, of a gold nanoparticle carrier comprising a double-stranded DNA that is bound to a surface thereof through a residue having one or more functionalities and that expresses insulin or expresses a substance that promotes insulin secretion.

## Claims

1. A gene carrier comprising: a gold nanoparticle; and a double-stranded DNA that is bound to the gold nanoparticle through a residue comprising one or more functionalities and, when delivered into a cell, independently expresses insulin or expresses a substance that promotes insulin secretion.

2. The gene carrier of claim 1, wherein the double-stranded DNA comprises an insulin gene (INS, Insulin gene).

3. The gene carrier of claim 1, wherein the substance that promotes insulin secretion comprises one selected from GLP-1 (Glucagon-Like Peptide-1), a GLP-1 receptor agonist (GLP-1 Receptor Agonists), GIP (Gastric Inhibitory Polypeptide), oxyntomodulin (Oxyntomodulin), PYY (Peptide YY), CCK (Cholecystokinin), a DPP-4 inhibitor (Dipeptidyl Peptidase-4 Inhibitors), and PDX1 (Pancreatic and Duodenal Homeobox 1).

4. The gene carrier of claim 1, wherein the double-stranded DNA is independently expressed without being integrated into the genome of the cell into which the double-stranded DNA is introduced.

5. The gene carrier of claim 1, wherein the residue comprising the functionality comprises a thiol group or an amine group.

6. The gene carrier of claim 1, wherein the residue comprising the functionality is one or more residues comprised at the 3' end, the 5' end, or within the nucleotide sequence of the double-stranded DNA.

7. The gene carrier of claim 1, wherein the gold nanoparticle has a size of 5 to 500 nm.

8. A pharmaceutical composition for the prevention or treatment of a metabolic disease, comprising the gene carrier of any one of claims 1 to 7.

9. The pharmaceutical composition of claim 8, wherein the metabolic disease is selected from the group consisting of diabetes, obesity, dyslipidemia, fatty liver disease, metabolic syndrome, and cardiovascular disease such as hypertension.

10. A method for the prevention or treatment of a metabolic disease using a gold nanoparticle carrier, the method comprising:
a step of binding, to a surface of a gold nanoparticle, a double-stranded DNA that expresses insulin or that expresses a substance that promotes insulin secretion, and introducing the gold nanoparticle into a cell; and
a step of independently expressing, within the nucleus, the double-stranded DNA bound to the surface of the gold nanoparticle.

11. A use, for the prevention or treatment of a metabolic disease, of a gold nanoparticle carrier comprising a double-stranded DNA that is bound to a surface thereof through a residue having one or more functionalities and that expresses insulin or expresses a substance that promotes insulin secretion.
